# EUROPEAN PATENT SPECIFICATION

(11) **EP 2 822 797 B1**
(45) Date of publication and mention of the grant of the patent: **24.01.2018**
(21) Application number: 12723260.1
(22) Date of filing: 06.03.2012
(51) Int. Cl.: B60K 28/06, A61B 5/18, A61B 5/00

(54) **REMOTE DETECTOR OF THE ALCOHOLIC, SMOKE OR& xA;OTHER CHEMICAL OR NATURAL PRODUCT LEVEL, NEGATIVELY& xA;IMPAIRING THE PSYCHOPHYSICAL STATE OF A PERSON DRIVING A& xA;VEHICLE**
FERNDETEKTOR FÜR ALKOHOL-, RAUCH- ODER ANDERE PEGEL CHEMISCHER ODER NATÜRLICHER DEN PHYSIOLOGISCHEN STATUS EINER PERSON BEIM FAHREN EINES FAHRZEUGS BEEINTRÄCHTIGENDER PRODUKTE
DÉTECTEUR À DISTANCE DU TAUX D'ALCOOL, DE FUMÉE OU D'UNE AUTRE SUBSTANCE CHIMIQUE OU D'UN PRODUIT NATUREL AFFECTANT NÉGATIVEMENT L'ÉTAT PSYCHOPHYSIQUE D'UNE PERSONNE CONDUISANT UN VÉHICULE

(43) Date of publication of application: 14.01.2015
(73) Proprietor: VIANELLO, Riccarda, 19037 Santo Stefano Magra (SP) (IT)
(72) Inventor: AZZARINI Antonio, 19037 Santo Stefano Megra (SP) (IT)
(74) Representative: Benelli, Cristian
(86) International application number: PCT/IT2012/000064
(87) International publication number: WO 2013/132521

(56) References cited:
- WO-A1-01/12457
- WO-A1-94/25945
- WO-A1-2010/097508
- DE-U1-202007 018 271
- JP-A- 2008 214 770
- US-A- 4 613 845
- US-A1- 2011 102 182

## Description

### Prior Art

DE 20 2007 018271 U1 discloses an apparatus for detecting the level of alcoholic level of a person driving a motor vehicle, of a type comprising: at least a detecting sensor to be installed on the motor vehicle; said sensor being connected to one or more outer relays in order to act on the motor vehicle electric circuit and to output a motor vehicle stop; said sensor being capable of constantly analysing the concentration of alcohol vapours and of signalling the results, a receiving sensor for acquiring and processing the signals from the detecting sensor.

### DESCRIPTION

It is a digital electro-chemical, mechanical device, of the "drunkometer" or gas detector type, but completely programmed and modified with reference to its components and parts; it is mounted on the driving-wheel, or another part of a motor vehicle or, more generally, a ground vehicle, an aircraft and a watercraft; the device "detects", by a digital analytical computerized sensor, at a distance between 30-40 cm, if the driver has a breath alcoholic level greater than the law allowed requirement.

Such device (which can be implemented also with rechargeable batteries) since is connected to the electric or electronic circuit of the vehicle, will trigger an electromechanical relay which, in turn, will drive an automatism (of the infra-red type or another kind of system) or a program which will prevent the vehicle from starting up.

The device will act on the fuel pump, by shutting off the fuel flow, or on the electric circuit outside the control unit, which supplies the current from the battery to the vehicle circuit.

The device will be capable of stopping cars which are already in motion, or which are stationary, if a second driver will take the place of a first drunk one.

The device, when is tampered or disconnected or covered, equally stops the car which cannot be started up.

The device can be delivered as a kit to be installed, as an option, and sold only through authorized retailers for cars or vehicles which are not provided with such device.

As an alternative to the "vehicle starting up prevention" feature, it is possible to activate a siren or flashing lights or vocal or optical warnings inside the vehicle, to warn the driver of his/her physical state, in order to desist he/she from driving.

Therefore, it is an automatic electronic-digital device, positioned adjacent, preferably in front of, the driver of the vehicle which is associated to, and it is capable of detecting the alcoholic level of the same, without the necessity of blowing in a tube or pipe connected to the detector.

In case of a detection of parameters greater than a threshold, the device does not enable to start up the vehicle; instead, the device can supply information (for example SMS or prerecorded vocal calls) to the company or family, to inform them that the vehicle cannot be started up because the alcoholic level of the driver is greater than the one specified by the law.

If the driver is drinking alcoholic substances while he/she is driving, the device, after having detected the alcoholic substance, sends a communication or a sound or vocal warning, or a SMS, in this case without stopping the vehicle.

Preferably, the invention is located in front of or laterally with respect to the driving position, so that the distance between the device and the driver is about 30-40 cm. The device is connected to a suitable bracket of the type used for the backward mirror; in addition, since the device can be disconnected from the bracket, the invention acts also as an anti-theft, because the disconnection from the bracket opens the electric circuit, which in turn prevents the vehicle from being started up.

### BRIEF DESCRIPTION OF THE DRAWINGS

This and other characteristics will be better understood from the following description of some embodiments shown in an exemplifying and non-limiting way in the attached drawings of a remote device for detecting the level of alcohol or another chemical or natural product, negatively impairing the psychophysical state of a person driving a vehicle.

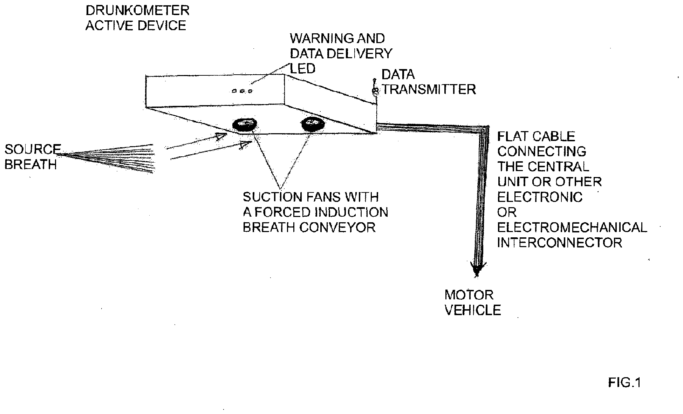

- Figure 1 is a operative schematic view of the transmitting-receiving apparatus,

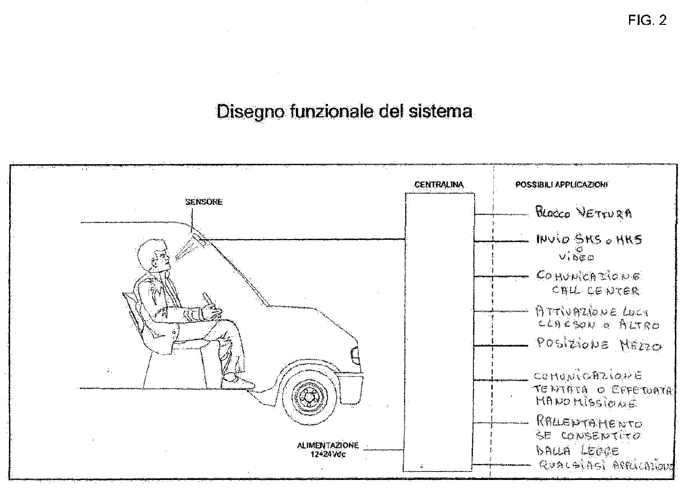

- Figure 2 is an overall view of the apparatus in a motor vehicle and connected to a central unit,

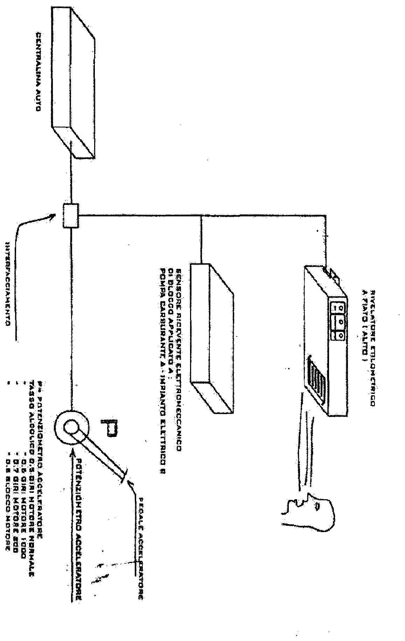

- Figure 3 is a variant of an embodiment wherein the detecting apparatus is located over the driver, and the receiving sensor is connected to the fuel pump or electric circuit, for connecting itself to the central unit and for stopping the motor vehicle in case of values greater than a threshold.

### DESCRIPTION OF THE DEVICE

With reference to the figures, 1 globally indicates the apparatus for detecting the alcoholic level to be installed in motor vehicles. It comprises an alcohol detector fixedly installed in a motor vehicle which enables to detect alcohol vapors emitted with the breath of the driver.

The detector can be also arranged to be used inside motorcycle helmets, in other words it is arranged in a position corresponding to the motorcyclist mouth, under the visor. In this case, the detector is based on a wireless communication, such as Bluetooth.

A receiving sensor, such as a central unit or a remote control element, is located in the motor vehicle, in a protected area under the dashboard of the vehicle.

This central unit is connected to one or more external relays which enable the horn, flashing lights, headlights, speed limiter, etcetera.

In other words, the receiving sensor is connected to the fuel pump or electric circuit of the vehicle in order to act on the same and generate consecutive warnings or the vehicle stop.

The invention provides the possibility of acquiring an analog signal for an environmental monitoring system which can record the alcoholic level of the driver, by acting as a kind of black box used for the aircrafts.

It is also possible to send enable or disable signals from a remote control safety center, which, after having advised the driver of the danger, can forcefully disable the vehicle in case of an excessive alcoholic level in the context of a public safety.

### OPERATIVE DESCRIPTION OF THE INVENTION

When the dashboard is turned on, the system cuts off the controls of the ignition key, in this way it does not enable both the turn on of the dashboard and the start-up of the vehicle.

During the start-up of the vehicle, said device firstly analyzes the air surrounding the driver and verifies if the alcohol vapors emitted are less than a predetermined threshold.

Said state of a first analysis is shown by a LED, preferably a yellow LED which shows to wait on the sensor panel.

After some seconds, the system turns off the LED by enabling the dashboard so that the driver can start up the vehicle.

The system, object of the invention, also when the vehicle is in motion, constantly analyzes the concentration of the alcoholic vapors inside the cabin and signals the analysis result.

In case it detects a percentage of the alcohol vapors in the air less than 0.5%, the system turns on only a LED activated on the sensor panel.

In case of a greater concentration, besides the above-mentioned optical message, there are:
- an intermittent turn on of the acoustic warning,
- a turn on of the four blinkers of the vehicle, if required,
- an activation of the speed limiter for reducing the vehicle speed to a minimum, if required.

In case the parameters are greater than the limit threshold, besides the above-mentioned advices, there are:
- the acoustic activation (horn) of the vehicle,
- the turn on of a further STOP LED,
- deactivation of the dashboard output for at least two seconds and possibly, in case of high concentrations, deactivation with reactivation of the ignition key rotation from the OFF position.

At this point, the system performs a new analysis and controls again the cabin alcoholic concentration.

The SPEED LIMITER output turns on an outer relay adapted to be connected to the accelerator potentiometer, which almost any vehicle is provided with, located along the connection cable between the potentiometer and the electronic central unit of the vehicle, for decreasing the vehicle speed to a minimum.

The BLINKER and CLACSON outputs are used for signaling to the preceding or following cars that the vehicle is going to stop, and also for drawing the attention of the police.

Other flags and programmable output signals are available at this connector, for example in case of an excessive alcoholic level, the same central unit, by optional outer interfaces, can send a SMS. signal containing the latitude and longitude of the GPS position of the vehicle, enabling in this way to track the same.

Besides that, the remote control center can forcedly perform possible disturb and dissuasive measures or as described beforehand, outwardly communications measures for asking for help, because the vehicles cannot be started up because the driver was disabled from doing it.

The described apparatus, and particularly the alcohol vapors detecting sensor, is installed inside the vehicle, and in the high area of the cabin, immediately above and in front of the driver, or preferably adjacent the mouth.

The sensor, whose software portion has been modified and reprogrammed, enables the device to sense the presence of smoke in the cabin or in an area adjacent the detector itself. In fact, it is remembered that in many European countries (for example United Kingdom and San Marino) it is strictly forbidden to smoke during the driving, also to a driver of a private car. In such case, the device does not stop the vehicle, but it sends a communication, such as a SMS, to the company or to a person who is responsible, by informing him/her about the perpetrated wrongdoing.

According to a further variant of an embodiment, not shown, the detecting device can be located inside the helmets for motorbikes and two-wheeled vehicles.

The drunkometer is provided with at least two micro-directional fans provided with suitable side deviating fins, capable of better collecting the molecules of the drawn substances.

The herein described device moreover comprises, or at least is provided for being connected to, means for acquiring images, such as for example a camera, in order to detect the driver state. The image acquisition can be done by positioning in a suitable area the camera or an equivalent means, preferably in front of a driver, for possible controls on the vehicle driving.

In addition, audible communication means, such as for example interphones, can be coupled in order to enable a fast conversation for example with the transport company, the family, etcetera.

Said remote apparatus can be coupled with a storage device, and also can be coupled with a device for printing the alcoholic level, the detection time and the position.

The device, emitting and/or receiving sensor, are connected to the associated motor vehicles by fast release systems which can automatically release themselves in case of an impact.

Further, the apparatus comprises:
- anti-intrusion control means adapted to short-circuit the system and prevent the start-up of the vehicle when the inlet opening of the detecting sensor is obstructed; said start-up prevention is caused by the closure of the one or more molecular suction fans; in such case, the system automatically sends a message (SMS, MMS) to the operative headquarters or to the family,
- a fast release device arranged behind the emitting device, adapted to release it in case there is an acceleration increase caused by an impact of the vehicle,
- warning and engine-shut-off means which can be operated in case the detector and/or the receiving sensor are tampered, acting as an anti-theft and opening the electric circuit of the motor vehicle.

Lastly, the described apparatus comprises one or more sensors arranged in the vehicle, on the driving wheel, on the handlebars, the shift, etcetera, adapted to detect the Ph of the skin of the driver by determining the presence of possible drugs and communicating this fact to the receiver which will intervene on the motor vehicle.

The remote apparatus for detecting the level of alcohol or another chemical or natural product, is capable of communicating with a central unit of the vehicle and activating, in case the read values are greater than threshold parameters:
- the lowering of the electric windows,
- the activation of the emergency lights,
- the activation of the acoustic warning.

Lastly, the apparatus, besides intervening on the central unit, can intervene also on the fuel flow.

Alternatively, the overall system can be deactivated or disconnected by a secret code (a key or a mobile phone) for a possible obvious assistance on the apparatus, or for specified safety reasons.

The device can comprise a port for communicating with outer apparatus (of the USB interface type), wherein the company, the family, or the police can download data associated to the driving.

## Claims

1. Apparatus for detecting the level of alcoholic, chemical or natural products negatively impairing the psychophysical state of a person driving a motor vehicle, of a type comprising:
a. at least a detecting sensor to be installed on the motor vehicle and comprising at least two micro-directional fans provided with suitable side deviating fins, capable of collecting the molecules of the drawn substances; said detecting sensor being connected to one or more outer relays enabling the horn, blinkers, headlights, speed limiter, and the detection sensor being connected to the motor vehicle electric circuit and/or the fuel pump to shut off the fuel flow, or the electric circuit outside the control unit, which supplies the current from the battery to the vehicle circuit and to generate consecutive warnings or the vehicle stop; said detecting sensor being capable of constantly analyzing the concentration of alcohol vapours and/or chemical or natural products, and of signalling the results,
b. a receiving sensor, such as a central unit or a remote control element, for acquiring and processing the signals from the detecting sensor comprising
- anti-intruder control means adapted to short-circuit the system and prevent the vehicle start-up if the inlet opening of the detecting sensor is obstructed; said stop occurs upon the closure of the one or more molecular suction fans;
- a fast release device arranged behind the detecting sensor and adapted to release it in case of an acceleration increase caused by an impact of the vehicle;
- warning and engine-shut-off means activable in case of a tamper of the connection between the apparatus and its bracket and/or a tamper of the receiving sensor, acting as an anti-theft feature and opening the electric circuit of the motor vehicle and preventing the vehicle from being started up.

2. Apparatus according to claim 1, further comprising one or more sensors arranged in the vehicle, on the driving wheel, on the handlebars, or on the shift, adapted to detect the Ph-value of the skin of the driver for determining the presence of possible drugs, their presence being communicated to the receiver which will intervene on the motor vehicle.

3. Method for operating an apparatus according to claim 1 and for detecting the level of alcohol or another chemical or natural product, negatively impairing the psychophysical state of a person, comprising the steps of:
a. upon the turn-on of the dashboard, the system cuts off the controls of the ignition key to disable the turn-on of the dashboard, and the start-up of the motor vehicle,
b. during the first start-up of the motor vehicle, said apparatus performs an analysis of the air surrounding the driver and verifies if the drawn alcohol vapors level is lower than a predetermined threshold, and said first analysis state is shown by a LED and by a possible stand-by caption on the sensor panel,
c. the processing systems verifies if the drawn alcohol vapors level is lower than the predetermined threshold and if this condition is satisfied, the system will turn off the LED and enable the dashboard to start up the motor vehicle and the apparatus communicating with a vehicle central unit and activating, in case of a reading greater than the threshold values of the relevant parameters:
a. the lowering of the electric windows,
b. the turn on of the emergency lights,
c. the turn on of the acoustic warning.

4. A vehicle comprising an apparatus according to 1 claim 1, wherein the detecting sensor is positioned in the upper portion of the cabin, immediately above and in front of the driver, preferably adjacent his/her mouth, while the central unit is arranged in a protected position under the vehicle dashboard.

5. A motor vehicle comprising an apparatus according to claim 1, wherein the detecting sensor is arranged inside an helmet adjacent the driver mouth.

6. A helmet comprising a detecting sensor of an apparatus according to claim 1 and that is adapted to interact with the apparatus of claim 1.

## Patentansprüche

1. Vorrichtung zur Erfassung des Niveaus von alkoholischen, chemischen oder natürlichen Produkten, die den psychophysischen Zustand einer Person, die ein Kraftfahrzeug fährt, negativ beeinflussen, von einem Typ umfassend:
a. mindestens ein Erfassungssensor, der an dem Kraftfahrzeug zu installieren ist, umfassend mindestens zwei mikrodirektionale Ventilatoren, die mit geeigneten seitlichen Ablenkflügeln versehen sind, die in der Lage sind, die Moleküle der angesaugten Substanzen zu sammeln; wobei der Erfassungssensor mit einem oder mehreren externen Relais verbunden ist, die es ermöglichen, dass an den Stromkreis des Kraftfahrzeuges angeschlossene Hörner, Blinklichter, Projektoren, Geschwindigkeitsbegrenzer und Erfassungssensoren und/oder die Kraftstoffpumpe den Kraftstofffluss oder die elektrische Schaltung außerhalb der Steuereinheit unterbrechen, die den Strom von der Batterie an die Fahrzeugschaltung liefern, um wiederholte Alarme zu erzeugen oder das Fahrzeug anzuhalten; wobei der Erfassungssensor ist in der Lage, ständig die Konzentration von Alkohol, Dämpfen und/oder chemischen oder natürlichen Produkten zu analysieren und die Ergebnisse zu melden,
b. ein Empfangssensor ist als eine zentrale Einheit oder ein Fernbedienungselement ausgebildet, um die Signale von dem Erfassungssensor zu erfassen und verarbeiten, umfassend:
- Einbruchsschutzkontrollmittel, die zum Kurzschließen des Systems und zum Verhindern des Startens des Fahrzeugs geeignet sind, wenn die Erfassungsöffnung des Erfassungssensors blockiert ist; wobei die Abschaltung erfolgt, sobald eine oder mehrere molekulare Saugflügel geschlossen sind;
- eine Schnelllösevorrichtung, die sich hinter dem Erfassungssensor befindet und dazu ausgelegt ist, diese im Falle einer Erhöhung der Beschleunigung aufgrund eines Aufpralls des Fahrzeugs zu lösen,
- Motoralarm- und Abschaltmittel, die im Falle eines Eindringens in die Verbindung zwischen dem Vorrichtung und seiner Halterung und/oder eines Eindringens in den Empfangssensor aktiviert werden können, als Diebstahlsicherung wirken und den Stromkreis des Kraftfahrzeugs derart öffnen, dass das Starten des Fahrzeugs verhindert wird.

2. Vorrichtung nach Anspruch 1, weiterhin umfassend einen oder mehrere Sensoren, die im Fahrzeug, am Lenkrad, am Lenker oder am Gangwechsel angeordnet sind und dazu eingerichtet sind, um den pH-Wert der Haut des Fahrers zu erfassen, um das Vorhandensein möglicher Drogen festzustellen; wobei dann dem Empfänger mitgeteilt wird, dass sie in das Fahrzeug eingreifen wird.

3. Verfahren zum Betreiben einer Vorrichtung nach Anspruch 1 und zum Erfassen des Niveaus an Alkohol oder anderen chemischen oder natürlichen Produkten, die den psychophysischen Zustand einer Person negativ beeinflussen, umfassend die Schritte:
a. nach dem Einschalten der Instrumententafel unterbricht das System die Steuerungen des Zündungsschlüssels, schaltet die Zündung der Instrumententafel aus und startet das Fahrzeug;
b. während der ersten Inbetriebnahme des Kraftfahrzeugs führt die Vorrichtung eine Analyse der Luft um den Fahrer herum durch und überprüft, ob das Niveau der angesaugten Alkoholdämpfe niedriger als ein vorbestimmter Grenzwert ist, und der erste Analysestatus wird durch eine LED und durch eine mögliche Schrift auf dem Sensortafel gezeigt,
c. die Verarbeitungssysteme überprüfen, ob der Niveau der angesaugten Alkoholdämpfe unter der vorgegebenen Grenze liegt. Wenn diese Bedingung erfüllt ist, schaltet das System die LED aus und ermöglicht dem Instrumententafel das Fahrzeug zu starten, und die Vorrichtung kommuniziert mit einer Zentraleinheit des Fahrzeugs und bei Überschreitung der Grenzwerte folgende wichtigen Parameter steuert:
a. Absenken der elektrischen Fensterheber,
b. Notleuchten einschalten,
c. Zündung des akustischen Alarms.

4. Fahrzeug mit einer Vorrichtung nach Anspruch 1, **dadurch gekennzeichnet, dass** der Detektionssensor im oberen Teil des Fahrgastraums unmittelbar oberhalb und vor dem Fahrer ist, benachbart zu seinem Mund, angeordnet ist, während die Zentraleinheit in einem geschützten Raum unter der Instrumententafel des Fahrzeugs angeordnet ist.

5. Kraftfahrzeug mit einer Vorrichtung nach Anspruch 1, **dadurch gekennzeichnet, dass** der Erfassungssensor innerhalb eines dem Fahrermund benachbarten Helms angeordnet ist.

6. Helm mit einem Erfassungssensor einer Vorrichtung nach Anspruch 1, der zum Zusammenwirken mit der Vorrichtung nach Anspruch 1 geeignet ist.

## Revendications

1. Dispositif pour détecter le niveau de produits alcooliques, chimiques ou naturels affectant négativement l'état psychophysique d'une personne conduisant un véhicule automobile, d'un type comprenant:
a. au moins un capteur de détection à installer sur le véhicule automobile et comprenant au moins deux ventilateurs micro-directionnels pourvus d'ailes déflectrices latérales adaptées, aptes à recueillir les molécules des substances aspirées; ledit capteur de détection étant connecté à un ou plusieurs relais externes qui permettent de connecter des klaxons, des feux clignotants, des projecteurs, des limiteurs de vitesse et des capteurs de détection au circuit électrique du véhicule automobile et/ou à la pompe à carburant pour interrompre le débit de carburant ou le circuit électrique à l'extérieur de l'unité de commande qui fournit le courant de la batterie au circuit du véhicule et pour générer des alarmes répétés ou pour arrêter le véhicule; ledit capteur de détection étant capable d'analyser en permanence la concentration d'alcool, de vapeurs et/ou de produits chimiques ou naturels, et de rapporter les résultats,
b. un capteur de réception en tant qu'unité centrale ou élément de télécommande, pour acquérir et traiter les signaux provenant du capteur de détection, comprenant:
- des moyens de commande anti-intrusion aptes à court-circuiter le système et à empêcher le démarrage du véhicule, si l'ouverture de détection du capteur de détection est obstruée; ledit arrêt a lieu une fois qu'une ou plusieurs ventilateurs d'aspiration moléculaires sont fermés;
- un dispositif de déverrouillage rapide situé derrière le capteur de détection et apte à le libérer en cas d'augmentation de l'accélération provoquée par un choc du véhicule,
- des moyens d'alarme et d'arrêt du moteur activables en cas d'intrusion dans la liaison entre l'appareil et son support et/ou une intrusion sur le capteur de réception agissant comme dispositif antivol et ouvrant le circuit électrique du véhicule automobile, et empêchant le démarrage du véhicule.

2. Dispositif selon la revendication 1, comprenant en outre un ou plusieurs capteurs disposés dans le véhicule, sur le volant, sur le guidon ou sur le changement de vitesse, adapté pour détecter le pH de la peau du conducteur afin de déterminer la présence de médicaments éventuels, leur présence étant ensuite communiquée au récepteur qui interviendra sur le véhicule.

3. Procédé pour faire fonctionner un dispositif selon la revendication 1 et pour détecter le niveau d'alcool ou d'autre produit chimique ou naturel qui affecte négativement l'état psychophysique d'une personne, comprenant les étapes consistant à:
a. après avoir allumé le tableau de bord, le système interrompt les commandes de la clé de contact, ce qui désactive l'allumage du tableau de bord et le démarrage du véhicule,
b. lors de la première mise en route du véhicule automobile, ledit dispositif effectue une analyse de l'air autour du conducteur et vérifie si le niveau des vapeurs d'alcool aspirées est inférieur à une limite prédéterminée, et ledit premier état d'analyse est indiqué par une LED et par un possible écrit sur le panneau du capteur,
c. les systèmes de traitement vérifient si le niveau des vapeurs d'alcool aspirées est inférieur à la limite prédéterminée, et si cette condition est remplie, le système éteint la LED et permet au tableau de bord de démarrer le véhicule, et le dispositif communique avec une unité centrale du véhicule et avec une activation, en cas d'une lecture au-dessus des valeurs limites des paramètres importants suivants:
a. abaissement des fenêtres électriques,
b. allumage des lumières de secours,
c. allumage de l'alarme acoustique.

4. Véhicule comprenant un dispositif selon la revendication 1, dans lequel le capteur de détection est disposé dans la partie supérieure de l'habitacle, immédiatement au-dessus et devant le conducteur, de préférence adjacent à sa bouche, tandis que l'unité centrale est disposée dans un position protégée sous le tableau de bord du véhicule.

5. Véhicule automobile comprenant un dispositif selon la revendication 1, dans lequel le capteur de détection est disposé à l'intérieur d'un casque adjacent à la bouche du conducteur.

6. Casque comprenant un capteur pour détecter un dispositif selon la revendication 1, apte à interagir avec le dispositif de la revendication 1.
